# EUROPEAN PATENT SPECIFICATION

(11) **EP 2 040 755 B1**
(45) Date of publication and mention of the grant of the patent: **18.05.2011**
(21) Application number: 07765553.8
(22) Date of filing: 22.06.2007
(51) Int. Cl.: A61K 45/06, A61K 31/4188, A61K 31/445, A61P 25/28, A61P 3/04

(54) **COMBINATION OF A CHOLINESTERASE INHIBITOR AND A COMPOUND WITH 5-HT6 RECEPTOR AFFINITY**
KOMBINATION AUS EINEM CHOLINESTERASE-HEMMER UND EINER VERBINDUNG MIT 5-HT6-REZEPTOR-AFFINITÄT
COMBINAISON D'UN INHIBITEUR DE LA CHOLINESTÉRASE ET D'UN COMPOSÉ AYANT UNE AFFINITÉ ENVERS LE RÉCEPTEUR 5-HT6

(30) Priority: 23.06.2006 EP 06384012
(43) Date of publication of application: 01.04.2009
(73) Proprietor: Laboratorios del. Dr. Esteve, S.A., 08041 Barcelona (ES)
(72) Inventor: CODONY-SOLER, Xavier, 08041 Barcelona (ES); BUSCHMANN, Helmut Heinrich, 08960 San Just Desvern (Barcelona) (ES)
(74) Representative: ABG Patentes, S.L.
(86) International application number: PCT/EP2007/056234
(87) International publication number: WO 2007/147883

(56) References cited:
- EP-A- 1 632 491
- HOLENZ ET AL: "Medicinal chemistry strategies to 5-HT6 receptor ligands as potential cognitive enhancers and antiobesity agents" DRUG DISCOVERY TODAY, ELSEVIER, RAHWAY, NJ, US, vol. 11, no. 7-8, April 2006 (2006-04), pages 283-299, XP005362935 ISSN: 1359-6446
- "S.28.05 The 5-HT6 receptor: memories are made of this" EUROPEAN NEUROPSYCHOPHARMACOLOGY, ELSEVIER SCIENCE PUBLISHERS BV, AMSTERDAM, NL, vol. 15, 2005, page S357, XP005556554 ISSN: 0924-977X
- PEREZ-GARCIA G ET AL: "Oral administration of the 5-HT6 receptor antagonists SB-357134 and SB-399885 improves memory formation in an autoshaping learning task" PHARMACOLOGY BIOCHEMISTRY AND BEHAVIOR, ELSEVIER, US, vol. 81, no. 3, July 2005 (2005-07), pages 673-682, XP004976821 ISSN: 0091-3057
- MITCHELL ET AL: "5-HT6 receptors: a novel target for cognitive enhancement" PHARMACOLOGY AND THERAPEUTICS, ELSEVIER, GB, vol. 108, no. 3, December 2005 (2005-12), pages 320-333, XP005186746 ISSN: 0163-7258
- MITCHELL ET AL: "BGC20-761, a novel tryptamine analog, enhances memory consolidation and reverses scopolamine-induced memory deficit in social and visuospatial memory tasks through a 5-HT6 receptor-mediated mechanism" NEUROPHARMACOLOGY, PERGAMON PRESS, OXFORD, GB, vol. 50, no. 4, March 2006 (2006-03), pages 412-420, XP005287695 ISSN: 0028-3908
- "P.5.005 Effectiveness of donepezil in the treatment of memory impairment after traumatic brain injury a systematic review" EUROPEAN NEUROPSYCHOPHARMACOLOGY, ELSEVIER SCIENCE PUBLISHERS BV, AMSTERDAM, NL, vol. 15, 2005, page S559, XP005557011 ISSN: 0924-977X
- AMATO M P: "Donepezil for memory impairment in multiple sclerosis" LANCET NEUROLOGY, LANCET PUBLISHING GROUP, LONDON, GB, vol. 4, no. 2, February 2005 (2005-02), pages 72-73, XP004874657 ISSN: 1474-4422
- "P.5.010 Effects of rivastigmine on learning and short memory retention in rats with hypoxia-induced model of amnesia" EUROPEAN NEUROPSYCHOPHARMACOLOGY, ELSEVIER SCIENCE PUBLISHERS BV, AMSTERDAM, NL, vol. 15, 2005, pages S561-S562, XP005557016 ISSN: 0924-977X
- "P.5.001 Long-term efficacy and safety of galantamine in outpatients with mild cognitive disorder" EUROPEAN NEUROPSYCHOPHARMACOLOGY, ELSEVIER SCIENCE PUBLISHERS BV, AMSTERDAM, NL, vol. 15, 2005, page S558, XP005557007 ISSN: 0924-977X

## Description

### Field of the invention

The present invention relates to an active substance combination comprising at least one compound with 5-HT₆ receptor affinity, and at least one cholinesterase inhibitor, a medicament comprising said active substance combination, and the use of said active substance combination for the manufacture of a medicament.

### Background of the invention

Cognitive and/or degenerative brain disorders are characterized clinically by progressive loss of memory, cognition, reasoning, judgement and emotional stability that gradually leads to profound mental deterioration and ultimately death. In an example of such disorders, Alzheimer's disease is a common cause of progressive mental failure (dementia) in aged humans and is believed to represent the fourth most common medical cause of death in the United States. In particular, Alzheimer's disease is associated with degeneration of cholinergic neurons in the basal forebrain that play a fundamental role in cognitive functions, including memory. Cognitive and/or degenerative brain disorders have been observed in varied races and ethnic groups world-wide and presents a major public health problem. These diseases are currently estimated to affect about two to three million individuals in the United States alone and the occurrence will · increase world-wide as the human life span increases.

Cognitive and/or degenerative brain disorders are incurable with presently used medications, however, the symptoms of these disorders can be alleviated by using compounds useful for treating cognitive disorders, in particular for treating Alzheimer's disease, such as donepezil, rivastigmine, galantamine and phenserine all of which act as acetylcholinesterase inhibitors.

All acetylcholinesterase inhibitors have serious drawbacks in that they produce undesirable side affects caused by their activity as acetylcholinesterase inhibitors. These undesirable side effects are related to their toxicity caused by their suppression of acetylcholinesterase. Due to the fact that acetylcholinesterase inhibitors, which are administered chronically, have a low therapeutic ratio (i.e. the ratio between toxicity and therapeutic effect) they produce a number of pathologic conditions associated with cholinergic under activity. Therefore due to the chronic nature of treatment for cognitive disorders it has long been desired to provide a medicament which is effective and does not produce the toxic side effects inherent in the use of acetylcholinesterase inhibitors and does not present undesired side effects such as nausea and vomiting and, thus, can be administered for long periods.

### Brief description of the invention

It was therefore an object of the present invention to provide a medicament suitable for the prophylaxis and/or treatment of disorders related to acetylcholinesterase, and to 5-HT₆ receptors, which preferably does not show the undesired side effects of the conventional compounds which act as cholinesterase inhibitors, or at least less frequent and/or less pronounced.

In particular, it was an object of the present invention to provide a medicament suitable for the prophylaxis and/or treatment of cognitive disorders, which preferably does not show the undesired side effects of the conventional medicaments for the prophylaxis and/or treatment of cognitive disorders, or at least less frequent and/or less pronounced.

Said object has been achieved by providing an active substance combination comprising
(A) at least one compound with 5-HT₆ receptor affinity, which is 6-chloro-N-(3-(2-(dimethylamino)ethyl)-1H-indol-5-yl) imidazo[2,1-b]thiazole-5-sulfonamide
   and
(B) at least one cholinesterase inhibitor, which is donezepil or a salt, racemate or enantiomer thereof.

Donepezil hydrochloride is sold under the brand name Aricept® as a medication to treat Alzheimer's disease. The respective compound donepezil hydrochloride has the code names BNAG and E-2020, respectively.

Another object of the present invention relates to an active substance combination as defined above for its use as a medicament.

A third object of the invention refers to the use of the combination as defined above for the manufacture of a medicament for simultaneous acetylcholinesterase inhibition and 5-HT6-receptor regulation.

Even another object of the invention relates to the use of the combination as defined above for the manufacture of a medicament for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

Finally, another object refers to a pharmaceutical formulation, characterized in that it comprises an active substance combination as defined above and optionally one or more pharmacologically acceptable adjuvants.

### Detailed description of the invention

It has surprisingly been found that the compounds with 5-HT_{B} receptor affinity and the compounds which act as cholinesterase inhibitors show a synergistic effect in their pharmacological activities. Consequently, the dose of the corresponding compounds may be reduced in comparison to the dose necessary for an individual administration of said compounds. In particular, the combination of an amount of compound with 5-HT₆ receptor affinity and an amount of cholinesterase inhibitor which both basically do not show any pharmacological activity in these amounts leads to an active substance combination of these compounds which shows a pharmacological activity when these amounts are administered in combination.

According to the invention it has also been fond that the action of a acetylcholinesterase inhibitor potentiates the action of the compound with 5-HT₆ receptor affinity, so the combination of a cholinesterase inhibitor and a compound with 5-HT₆ receptor affinity for use in the treatment of disorders that are related to acetylcholinesterase, and to 5-HT₆ receptors may result in a faster onset of action and an increased success rate. The invention therefore resides in the combined action of a cholinesterase inhibitor and a compound with 5-HT₆ receptor affinity, or the dual action of a substance possessing both cholinesterase inhibitor activity and 5-HT₆ receptor affinity, for the treatment of disorders that are related to acetylcholinesterase, and to 5-HT₆ receptors.

The compound 6-chloro-N-(3-(2-(dimethylamino)ethyl)-1H-indol-5-yl) imidazo [2,1-b]thiazole-5-sulfonamide [1140] (also known as E-6801) can be prepared according to the disclosure of WO 2003/042175, WO 2005/013977 and WO 2006/024535. The respective parts of the literature are hereby incorporated by reference and form part of the present disclosure.

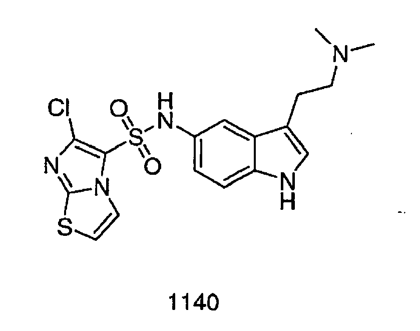

Preferably donepezil hydrochloride is present as component (B).

Particularly preferred is an active substance combination that comprises 1140 as component (A) and donepezil hydrochloride as component (B).

"Obesity" is a condition in which there is an excess of body fat. The operational definition of obesity is based on the Body Mass Index(BMI), which is calculated as body weight per height in meters squared (kg/m²). "Obesity" refers to a condition whereby an otherwise healthy subject has a Body Mass Index (BMI) greater than or equal to 30kg/m², or a condition whereby a subject with at least one co-morbidity has a BMI greater than or equal to 27kg/m². An "obese subject" is an otherwise healthy subject with a Body Mass Index (BMI) greater than or equal to 30 kg/m² or a subject with at least one co-morbidity with a BMI greater than or equal to 27 kg/m². A "subject at risk of obesity" is an otherwise healthy subject with a BMI of 25 kg/m² to less than 30 kg/m² or a subject with at least one co-morbidity with a BMI of 25 kg/m² to less than 27 kg/m²_{.}

The increased risks associated with obesity occur at a lower Body Mass Index(BMI) in Asians. In Asian countries, including Japan, "obesity" refers to a condition whereby a subject with at least one obesity-induced or obesity-related co-morbidity, that requires weight reduction or that would be improved by weight reduction, has a BMI greater than or equal to 25 kg/m². In Asian countries, including Japan, an "obese subject" refers to a subject with at least one obesity-induced or obesity-related co-morbidity that requires weight reduction or that would be improved by weight reduction, with a BMI greater than or equal to 25kg/m². in Asia-Pacific, a "subject at risk of obesity" is a subject with a BMI of greater than 23 kg/m² to less than 25 kg/m².

As used herein, the term "obesity" is meant to encompass all of the above definitions of obesity.

Obesity-induced or obesity-related co-morbidities include, but are not limited to, diabetes, non-insulin dependent diabetes mellitus-type II (2), impaired glucose tolerance, impaired fasting glucose, insulin resistance syndrome, dyslipidemia, hypertension, hyperuricacidemia, gout, coronary artery disease, myocardial infarction, angina pectoris, sleep apnea syndrome, Pickwickian syndrome, fatty liver; cerebral infarction, cerebral thrombosis, transient ischemic attack, orthopedic disorders, arthritis deformans, lumbodynia, emmeniopathy, and infertility.

In particular, co-morbidities include: hypertension, hyperlipidemia, dyslipidemia, glucose intolerance, cardiovascular disease, sleep apnea, diabetes mellitus, and other obesity-related conditions.

The term "cognitive disorder" indicates disruptions in performance including one or more of the following signs:
1) memory deficits (impaired ability to learn new information or recall previously learned information;
2) one (or more) of the following disturbances:
   a) aphasia (language disturbance)
   b) apraxia (impaired ability to carry out motor activities despite intact motor function)
   c) agnosia (failure to recognize or identify objects despite in tact sensory function)
   d) disturbance in executive functioning (i.e. planning, organizing, sequencing, abstracting);
3) memory disturbances causing significant impairment in social or occupational functioning, and representing a significant decline from a previous level of functioning; and
4) impairment in cognitive functioning as evidenced by neuropsychological testing or quantified clinical assessment, accompanied by objective evidence of a systemic general medical condition or central nervous system dysfunction.

Cognitive disorders may include Alzheimer's disease, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder.

"Treatment" (of obesity and obesity-related disorders) refers to the administration of the compounds or combinations of the present invention to reduce or maintain the body weight of an obese subject. One outcome of treatment may be reducing the body weight of an obese subject relative to that subject's body weight immediately before the administration of the compounds or combinations of the present invention. Another outcome of treatment may be preventing body weight regain of body weight previously lost as a result of diet, exercise, or pharmacotherapy.

Another outcome of treatment may be decreasing the occurrence of and/or the severity of obesity-related diseases. Another outcome of treatment may be to maintain weight loss. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate; and in weight reduction in patients in need thereof. The treatment may also result in an alteration of metabolic rate, such as an increase in metabolic rate, rather than or in addition to an inhibition of the reduction of metabolic rate; and/or in minimization of the metabolic resistance that normally results from weight loss.

The term "Metabolic syndrome", also known as syndrome X, is defined in the Third Report of the National Cholesterol Education Program Expert Panel on Detection, Evaluation and Treatment of High Blood Cholesterol in Adults (ATP-E). E. S. Ford et al. JAMA, Vol. 287 (3), Jan. 16, 2002, pp 356-359. Briefly, a person is defined as having Metabolic syndrome if the person has three or more of the following symptoms: abdominal obesity, hypertriglyceridemia, low HDL cholesterol, high blood pressure, and high fasting plasma glucose.

The terms "administration of" and or "administering a" compound should be understood to mean providing a compound of the invention or a prodrug of a compound of the invention to a subject in need of treatment.

The instant pharmaceutical composition includes administration of a single pharmaceutical dosage formulation which contains both the compound with 5-HT₆ receptor affinity, and at least one cholinesterase inhibitor, as well as administration of each active agent in its own separate pharmaceutical dosage formulation. Where separate dosage formulations are used, the individual components of the composition can be administered at essentially the same time, i. e. , concurrently, or at separately staggered times, i. e. sequentially prior to or subsequent to the administration of the other component of the composition. The instant pharmaceutical composition is therefore to be understood to include all such regimes of simultaneous or alternating treatment, and the terms "administration" and "administering" are to be interpreted accordingly.

Administration in these various ways are suitable for the present compositions as long as the beneficial pharmaceutical effect of the combination of the compound with 5-HT₆ receptor affinity, and at least one cholinesterase inhibitor, is realised by the patient at substantially the same time.

Such beneficial effect is preferably achieved when the target blood level concentrations of each active drug are maintained at substantially the same time. It is preferred that the combination of the compound with 5-HT₆ receptor affinity, and at least one cholinesterase inhibitor, be co-administered concurrently on a once-a-day dosing schedule; however, varying dosing schedules, such as the compound with 5-HT₆ receptor affinity once a day and the cholinesterase inhibitor once, twice or more times per day, is also encompassed herein. A single oral dosage formulation comprised of both a compound with 5-HT₆ receptor affinity and a cholinesterase inhibitor is preferred. A single dosage formulation will provide convenience for the patient, which is an important consideration especially for patients with diabetes or obese patients who may be in need of multiple medications.

The term "subject" as used herein refers to an animal, preferably a mammal, most preferably a human, who has been the object of treatment, observation or experiment. The administration of the composition of the present invention in order to practice the present methods of therapy is carried out by administering a therapeutically effective amount of the compounds in the composition to a subject in need of such treatment or prophylaxis. The need for a prophylactic administration according to the methods of the present invention is determined via the use of well known risk factors. The effective amount of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgement.

The term "therapeutically effective amount" as used herein means the amount of the active compounds in the composition that will elicit the biological or medical response in a tissue, system, subject, or human that is being sought by the researcher, veterinarian, medical doctor or other clinician, which includes alleviation of the symptoms of the disorder being treated. The novel methods of treatment of this invention are for disorders known to those skilled in the art.

The term "salt" as used herein is to be understood as meaning any form of the compounds in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, salicylic acid, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with NH₄.

Solvates, preferably hydrates, of the compounds of the present invention and in each case of corresponding stereoisomers may also be obtained by standard procedures known to those skilled in the art.

The term "solvate" according to this invention is to be understood as meaning any form of the compounds in which they have attached to it via non-covalent binding another molecule (most likely a polar solvent) especially including hydrates and alcoholates, e.g. methanolate.

The active substance combination according to this invention comprises preferably 1 - 99 % by weight of component (A) and 99 - 1 % by weight of component (B), more preferably 70 to 95 % by weight of component (A) and 30 to 5 % by weight of component (B), even more preferably 85 to 95 % by weight of component (A) and 15 to 5 % by weight of component (B), in each case referring to the total weight of both components (A) and (B)..

Another aspect of the present invention is the active substance combination of the invention for its use as a medicament.

Another aspect of the present invention is the use of an inventive active substance combination as defined above for the manufacture of a medicament for simultaneous acetylcholinesterase inhibition and 5-HT₆-receptor regulation.

Another aspect of the present invention is the use of the inventive active substance combination for the manufacture of a medicament for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Peripheral Nervous System Disorders, Central Nervous System Disorders, arthritis, epilepsy, anxiety, panic, depression, preferably bipolar disorders, cognitive disorders, memory disorders, cardiovascular diseases, senile dementia processes, neurodegenerative disorders, preferably Alzheimer's disease, Parkinson's diesease, Huntington's Disease and/or multiple sclerosis, schizophrenia, psychosis, infantile hyperkinesia (ADHD, attention deficit / hyperactivity disorder), pain, hypertensive syndrome, inflammatoric diseases, immunologic diseases or for improvement of cognition.

Particularly preferred is the use of the inventive active substance combination for the manufacture of a medicament for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome.

Also particularly preferred is the use of the inventive active substance combination for the manufacture of a medicament for prophylaxis and/or treatment of cognitive disorders, memory disorders, or senile dementia processes, such as Alzheimer's, Parkinson's and/or Huntington's Disease.

More particularly preferred is the use of the inventive active substance combination for the manufacture of a medicament for prophylaxis and/or treatment of obesity, cognitive disorders, memory disorders, or senile dementia processes, such as Alzheimer's, Parkinson's and/or Huntington's Disease.

Also particularly preferred is the use of the active substance combination for the manufacture of a medicament for the prophylaxis and/or treatment of obesity-related disorders such as elevated plasma insulin concentrations and insulin resistance, dyslipidemias, hyperlipidemia, endometrial, breast, prostate and colon cancer, osteoarthritis, obstructive sleep apnea, cholelithiasis, gallstones, heart disease, abnormal heart rhythms and arrythmias, myocardial infarction, congestive heart failure, coronary heart disease, sudden death, stroke, polycystic ovary disease, craniopharyngioma, the Prader-Willi Syndrome and Frohlich's syndrome. Further examples of obesity-related disorders are reproductive hormone abnormalities, sexual and reproductive dysfunction, such as impaired fertility, infertility, hypogonadism in males and hirsutism in females, fetal defects associated with maternal obesity, gastrointestinal motility disorders, such as obesity-related gastro-esophageal reflux, respiratory disorders, such as obesity-related hypoventilation syndrome (Pickwickian syndrome), breathlessness, cardiovascular disorders, inflammation, such as systemic inflammation of the vasculature, arteriosclerosis, hypercholesterolemia, hyperuricaemia, lower back pain, gallbladder disease, gout, kidney cancer, and increased anesthetic risk.

Those skilled in the art understand that the components (A) and (B) of the active substance combination according to the present invention may be administered simultaneously or sequentially to one another, whereby in each case components (A) and (B) may be administered via the same or different administration pathways, e.g. orally or parentally. preferably both components (A) and (B) are administered simultaneously in one and the same administration form.

Yet another aspect of the present invention are pharmaceutical formulations in different pharmaceutical forms comprising an inventive active substance combination and optionally one or more pharmacologically acceptable adjuvants.

As well known to somebody skilled in the art the pharmaceutical formulations may - depending on their route of administration, also contain one or more auxiliary substances known to those skilled in the art.

The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the disclosure.

Preferred pharmaceutical formulations are solid pharmaceutical forms, preferably tablets, chewing tablets, chewing gums, dragées, capsules, suppositories, powder preparations, transdermal therapeutic systems, transmucosal therapeutic systems, preferably tablets or capsules.

Preferred pharmaceutical formulations are also liquid and semi-liquid pharmaceutical forms such as drops or such as juice, sirup, solution, emulsion, suspension, preferably drops or solutions.

In an additional preferred embodiment, the pharmaceutical formulations are in the form of multiparticulates, preferably microtablets, microcapsules, microspheroids, granules, crystals or pellets, optionally compacted in a tablet, filled in a capsule or suspended in a suitable liquid.

The pharmaceutical formulations according to the present invention are particularly preferably suitable for oral, intravenous, intramuscular, subcutaneous, intrathecal, epidural, buccal, sublingual, pulmonal, rectal, transdermal, nasal or intracerebroventricular application, more particularly for oral, intravenous or intraperitoneal application.

In one embodiment of the present invention the pharmaceutical formulation comprises at least one of the components (A) and (B) of the active substance combination at least partially in a sustained-release form.

By incorporating one or both of these components (A) and (B) at least partially or completely in a sustained-release form it is possible to extend the duration of their effect, allowing for the beneficial effects of such a sustained-release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁₋₄)alkyl (meth)acrylates, poly(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D^{®}), copolymers of ethyl acrylate, methyl methacrylate and trimethylammonium ethyl methacrylate-chloride with a monomer molar ratio of 1:2:0.1 (Eudragit RS^{®}), copolymers of ethyl acrylate, methyl methacrylate and trimethylammonium ethyl methacrylate-chloride with a monomer molar ratio of 1:2:0.2 (Eudragit RL^{®}), or a mixture of at least two of the above-mentioned copolymers. These coating materials are commercially available as 30 wt.% aqueous latex dispersions, i.e. as Eudragit RS30D^{®}, Eudragit NE30D^{®} or Eudragit RL30D^{®}, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat^{®} or Surelease^{®}.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet^{®} (acetylated mono- and diglycerides, C₂₃H₄₄O₅ to C₂₅H₄₇O₇), medium-chain triglycerides (Migiyol^{®}), oleic acid or mixtures of at least two of said plasticizers.

Aqueous dispersions of Eudragit RS^{®} and optionally Eudragit RL^{®} preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L^{®}), methacrylic acid/methyl methacrylate copolymers with a monomer molar ratio of 1:2 (Eudragit S^{®}), methacrylic acid/ethyl acrylate copolymers with a monomer molar ratio of 1:1 (Eudragit L30D-55^{®}), methacrylic acid/methyl acrylate/methyl methacrylate copolymers with a monomer molar ratio of 7:3:1 (Eudragit FS^{®}), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D^{®} and/or Eudragit RL^{®} and/or Eudragit RS^{®}.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the disclosure.

In another embodiment, the pharmaceutical formulation of the present invention contains one or both of components (A) and (B) not only in sustained-release form, but also in non-sustained-release form. By combination with the immediately released form, a high initial dose can be achieved for the rapid onset of the beneficial effect. The slow release from the sustained-release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

Administered dosages for acetylcholinesterase inhibitors and compounds with 5-HT₆ receptor affinity are in accordance with dosages and scheduling regimens practised by those of skill in the art. General guidance for appropriate dosages of all pharmacological agents used in the present methods is provided in Goodman and Gilman's The Pharmacological Basis of Therapeutics, 10th Ed., Hardman, Limbird and Goodman-Gilman, Eds., McGraw-Hill (2001) and in a Physicians' Desk Reference (PDR), for instance, in the 57th or 58th Eds., Thomson PDR (2003 or 2004). Published dosages of acetylcholinesterase inhibitors and compounds for with 5-HT₆ receptor affinity are for indications distinct from treatment of obesity. Typically, efficacious dosages of acetylcholinesterase inhibitors and compounds with 5-HT₆ receptor affinity for practising the present invention can be equal to or less than (e.g., about 25, 50, 75 or 100%) the dosages published for other indications, such as for Alzheimer's disease and depression, respectively.

The present invention also relates to the treatment the aforementioned disorders and/or diseases with a combination of at least one compound with 5-HT₆ receptor affinity and at least one cholinesterase inhibitor which may be administered separately, therefore the invention also relates to combining separate pharmaceutical compositions into a kit form. The kit, according to this invention, comprises two separate pharmaceutical compositions: a first unit dosage form comprising a prophylactically or therapeutically effective amount of at least one cholinesterase inhibitor, or a pharmaceutically acceptable salt or ester thereof, and a pharmaceutically acceptable carrier or diluent in a first unit dosage form, and a second unit dosage form comprising a prophylactically or therapeutically effective amount of at least one compound with 5-HT₆ receptor affinity, or a pharmaceutically acceptable salt or ester thereof, and a pharmaceutically acceptable carrier or diluent in a second unit dosage form. The kit further comprises a container. Such kits are especially suited for the delivery of solid oral forms such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days or time in the treatment schedule in which the dosages can be administered.

### Examples

### Pharmacological methods:

### I) Binding to Serotonin receptor 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268,1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II) Behavioural Assessment of Cognitive effects of compounds with 5-HT₆ receptor affinity: Novel object discrimination (NOD)

Two separate groups of 12 adult male Lister hooded rats are tested in the NOD paradigm using 4 h inter-trial interval (ITI) delay between the familiarisation and choice trials which should ensure impaired discrimination and permit the pro-cognitive effects of the compounds to be evaluated.

In two separate groups of animals compound [1140] (6-chloro-N-(3-(2-(dimethylamino)ethyl)-1H-indol-5-yl)imidazo[2,1-b]thiazole-5-sulfonamide) and donepezil HCl (or vehicle) were administered on four separate occasions, at seven day intervals. The semi-randomised design for drug administration ensures that for each group, each animal receives one of the four treatment combinations once during the study: compound [1140] and donepezil HCl, compound [1140] and vehicle, donepezil HCl and vehicle, vehicle and vehicle. Hence, each animal will serve as its own control within each group to minimise inter-individual variability. Drug pre-treatment occurred 20 min prior to the first NOD trial to ensure optimal drug plasma levels during acquisition and consolidation phases.

To maximise the opportunity to see a synergistic effect, the animals received one dose of donepezil HCl i.p. (0.05 mg/kg for group 1 and 0.1 mg/kg for group 2) in combination with the selected dose of compound [1140] (1 mg/kg) or the vehicle (saline containing 0.5 methylcellulose, 2 ml/kg). The synergism between compound [1140] and donepezil HCl was studied at both 0.05 and 0.1 mg/kg i.p. At the end of the study, seven days after the last drug administration all rats were given donepezil HCl at 0.3 mg/kg i.p. to confirm reversal of natural forgetting and hence the animals were tested in the NOD paradigm at five occasions.

The exact experimental protocol is outlined in detail in:
M. V. King et al, 5-HT6 receptor antagonist reverse delay-dependent deficits in novel object discrimination by enhancing consolidation - an effect sensitive to NMDA receptor antagonism, Neuropharmacology 2004, 47, 195-204 and Woolley et al. Reversal of cholinergic-induced deficit in a rodent model of recognition memory by the selective 5-HT6 receptor antagonist, Ro 04-6790; Psychopharmacology 2003, 170, 358-367.

The respective parts of the literature are hereby incorporated by reference and form part of the present disclosure.

### Pharmacological data:

The behavioural assessment of cognitive effects of compounds with 5-HT₆ receptor affinity with the novel object discrimination (NOD) test was carried out as described above.

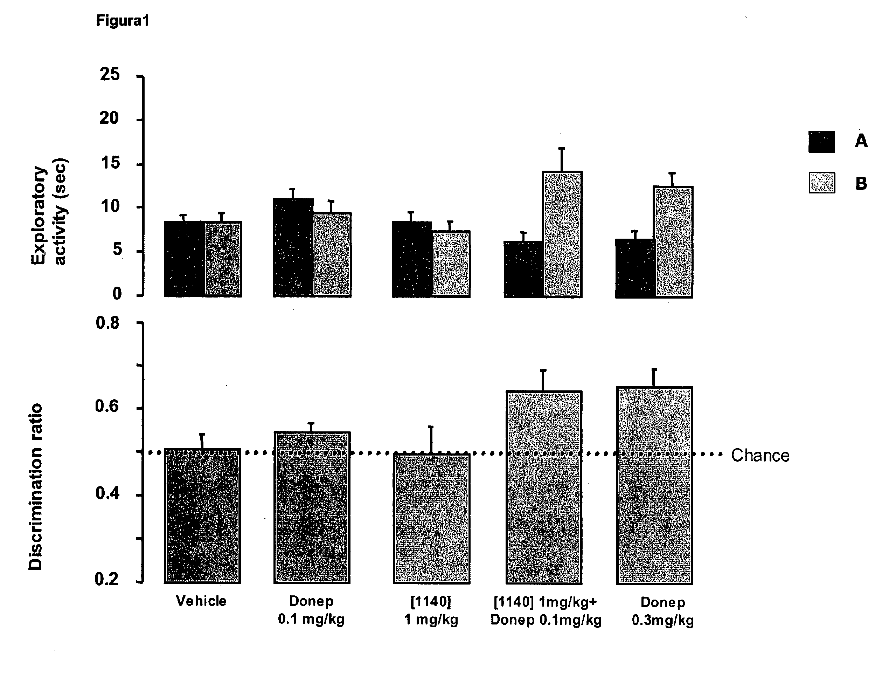

The results are depicted in figure 1. A denotes familiar and B denotes novel objects in this figure.

Figure 1. shows that the acetylcholinesterase inhibitor donepezil potentiates the action of the compound with 5-HT₆ receptor affinity, compound [1140]. Thus, the discrimination ratio is significantly greater when compound [1140] (1 mg/kg) is administered in combination with donepezil hydrochloride [compound [1140] (1 mg/kg) + donepezil hydrochloride(0.1 mg/kg)]. Figure 1. also shows that the combination of an amount of donepezil hydrochloride which does not have any effect in the NOD test by itself and an amount of compound [1140] which does not have any effect in the NOD test by itself can be combined to yield an active substance combination which shows a significant effect in the NOD test.

## Claims

1. An active substance combination that comprises:
(A) 6-chloro-N-(3-(2-(dimethylamino)ethyl)-1H-indol-5-yl)imidazo[2,1-b]thiazole-5-sulfonamide,
and
(B) donepezil or a salt, racemate or enantiomer thereof.

2. The combination according to claim 1, **characterized in that** the component (B) is donepezil hydrochloride.

3. The combination according to one or more of claims 1 to 2, **characterized in that** it comprises 1 - 99 % by weight of component (A) and 99 - 1 % by weight of component (B), preferably 70 to 95 % by weight of component (A) and 30 to 5 % by weight of component (B), more preferably 85 to 95 % by weight of component (A) and 15 to 5 % by weight of component (B), in each case referring to the total weight of both components (A) and (B).

4. An active substance combination as defined in anyone of claims 1 to 3 for its use as a medicament.

5. An active substance combination as defined in anyone of claims 1 to 3 for its use as a medicament for simultaneous acetylcholinesterase inhibition and 5-HT₆-receptor regulation.

6. An active substance combination as defined in anyone of claims 1 to 3 for its use as a medicament for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

7. A pharmaceutical formulation, **characterized in that** it comprises an active substance combination according to one or more of claims 1 to 3 and optionally one or more pharmacologically acceptable adjuvants.

8. The pharmaceutical formulation according to claim 7, **characterized in that** it is present in solid pharmaceutical forms such as tablets, chewing tablets, chewing gums, dragées, capsules, suppositories, powder preparations, transdermal therapeutic systems, transmucosal therapeutic systems, or in liquid and semi-liquid pharmaceutical forms such as drops or such as juice, sirup, solution, emulsion, suspension, preferably in form of tablets, capsules, drops or solution; or in form of multiple particles, preferably microtablets, microcapsules, microspheroids, granules, crystals or pellets, optionally compacted in a tablet, filled in a capsule or suspended in a suitable liquid.

9. The pharmaceutical formulation according to claims 7 or 8, **characterized in that** it is for oral, intravenous, intramuscular, subcutaneous, intrathecal, epidural, buccal, sublingual, pulmonal, rectal, transdermal, nasal or intracerebroventricular application, preferably oral or intravenous.

10. The pharmaceutical formulation according to claim 7 or 8, **characterized in that** at least one of the components of the active substance combination (A) or (B) is present at least partially in sustained-release form.

11. The pharmaceutical formulation according to claim 10, **characterized in that** the medicament has at least one coating or at least one matrix comprising at least one sustained-release material, preferably the sustained-release material is based on optionally modified, water-insoluble, natural, semisynthetic or synthetic polymer, or a natural wax or fat or fatty alcohol or semisynthetic or synthetic fatty acid, or on a mixture of at least two of these afore mentioned components.

12. The pharmaceutical formulation according to claim 11, **characterized in that** the water-insoluble polymer is based on an acrylic resin, which is preferably selected from the group of poly(meth)acrylates, poly(C₁₋₄)dialkylamino(C₁₋₄)alkyl (meth)acrylates and/or copolymers thereof or a mixture of at least two of the afore-mentioned polymers and/or the water-insoluble polymers are cellulose derivatives, preferably alkyl cellulose and even more preferably ethyl cellulose, or cellulose esters and/or the wax is carnauba wax, beeswax, glycerol monostearate, glycerol monobehenate, glycerol ditripalmitostearate, microcrystalline wax or a mixture of at least two of these components.

13. The pharmaceutical formulation according to one or more of claims 11 to 12, **characterized in that** polymers have been used in combination with one or more plasticizers.

14. The pharmaceutical formulation according to one or more of claims 10 to 13, **characterized in that** besides the sustained-release form, at least one of the active substance components (A) or (B) is present in a non-sustained-release form.

## Patentansprüche

1. Wirkstoffkombination, umfassend:
(A) 6-Chlor-N-(3-(2-(dimethylamino)ethyl)-1H-indol-5-yl)imidazo[2,1-b]thiazol-5- sulfonamid,
und
(B) Donepezil oder ein Salz, Racemat oder Enantiomer davon.

2. Kombination nach Anspruch 1, **dadurch gekennzeichnet, dass** der Bestandteil (B) Donepezil-Hydrochlorid ist.

3. Kombination nach einem oder mehreren der Ansprüche 1 bis 2, **dadurch gekennzeichnet, dass** die Kombination 1-99 Gew.-% des Bestandteils (A) und 99-1 Gew.-% des Bestandteils (B), vorzugsweise 70 bis 95 Gew.-% des Bestandteils (A) und 30 bis 5 Gew.-% des Bestandteils (B), besonders bevorzugt 85 bis 95 Gew.-% des Bestandteils (A) und 15 bis 5 Gew.-% des Bestandteils (B) umfasst, jeweils bezogen auf das Gesamtgewicht der beiden Bestandteile (A) und (B).

4. Wirkstoffkombination gemäß den Angaben in einem der Ansprüche 1 bis 3 zur Verwendung als Medikament.

5. Wirkstoffkombination gemäß den Angaben in einem der Ansprüche 1 bis 3 zur Verwendung als Medikament zur gleichzeitigen Acetylcholinesterase-Inhibierung und 5-HT₆-Rezeptor-Regulation.

6. Wirkstoffkombination gemäß den Angaben in einem der Ansprüche 1 bis 3 zur Verwendung als Medikament zur Regulierung des Appetits, zur Aufrechterhaltung, Erhöhung oder Verringerung des Körpergewichts, zur Prophylaxe und/oder zur Behandlung von Störungen, die im Zusammenhang mit Nahrungsaufnahme stehen, vorzugsweise zur Prophylaxe und/oder Behandlung von Adipositas, Anorexie, Kachexie, Bulimie, Diabetes, vorzugsweise Typ II-Diabetes (nicht-insulinabhängiger Diabetes mellitus), oder zur Prophylaxe und/oder Behandlung von Störungen des Gastrointestinaltrakts, vorzugsweise des Reizdarmsyndroms, zur Prophylaxe und/oder Behandlung von Metabolischem Syndrom, Störungen des peripheren Nervensystems, Störungen des Zentralnervensystems, Arthritis, Epilepsie, Angst, Panik, Depression, kognitiven Störungen, Gedächtnisstörungen, kardiovaskulären Erkrankungen, Prozessen seniler Demenz, wie beispielsweise Alzheimer-Krankheit, Parkinson-Krankheit und/oder Huntington-Krankheit, Schizophrenie, Psychose, infantiler Hyperkinese (ADHD, Aufmerksamkeitsdefizits- / Hyperaktivitätsstörung), Schmerz, Hypertensivem Syndrom, entzündlichen Erkrankungen, immunologischen Erkrankungen oder zur Verbesserung der Kognition.

7. Pharmazeutische Formulierung, **dadurch gekennzeichnet, dass** sie eine Wirkstoffkombination nach einem oder mehreren der Ansprüche 1 bis 3 und gegebenenfalls ein oder mehrere pharmakologisch akzeptable Adjuvantien umfasst.

8. Pharmazeutische Formulierung nach Anspruch 7, **dadurch gekennzeichnet, dass** sie vorliegt in festen pharmazeutischen Formen, wie beispielsweise Tabletten, Kautabletten, Kaugummis, Dragees, Kapseln, Zäpfchen, Pulverzubereitungen, transdermalen therapeutischen Systemen, transmukosalen therapeutischen Systemen, oder in flüssigen oder halbflüssigen pharmazeutischen Formen wie beispielsweise Tropfen oder wie Saft, Sirup, Lösung, Emulsion, Suspension, vorzugsweise in Form von Tabletten, Kapseln, Tropfen oder Lösung; oder in Form von Mehrfachpartikeln, vorzugsweise Mikrotabletten, Mikrokapseln, Mikrospheroiden, Granula, Kristallen oder Pellets, gegebenenfalls verpresst in einer Tablette, gefüllt in eine Kapsel oder suspendiert in einer geeigneten Flüssigkeit.

9. Pharmazeutische Formulierung nach den Ansprüchen 7 oder 8, **dadurch gekennzeichnet, dass** die pharmazeutische Formulierung für orale, intravenöse, intramuskuläre, subkutane, intrathekale, epidurale, bukale, sublinguale, pulmonale, rektale, transdermale, nasale oder intrazerebroventrikuläre Verabreichung ist, vorzugsweise für orale oder intravenöse Verabreichung.

10. Pharmazeutische Formulierung nach Anspruch 7 oder 8, **dadurch gekennzeichnet, dass** wenigstens einer der Bestandteile der Wirkstoffkombination (A) oder (B) wenigstens teilweise in einer Retard-Form vorliegt.

11. Pharmazeutische Formulierung nach Anspruch 10, **dadurch gekennzeichnet, dass** das Medikament wenigstens eine Beschichtung oder wenigstens eine Matrix aufweist, die wenigstens ein Retard-Material umfasst, wobei das Retard-Material vorzugsweise basiert auf einem gegebenenfalls modifizierten, wasserunlöslichen, natürlichen, halbsynthetischen oder synthetischen Polymer, oder einem natürlichen Wachs oder Fett oder Fettalkohol oder einer halbsynthetischen oder synthetischen Fettsäure, oder auf einem Gemisch von wenigstens zwei der zuvor genannten Bestandteile.

12. Pharmazeutische Formulierung nach Anspruch 11, **dadurch gekennzeichnet, dass** das wasserunlösliche Polymer basiert auf einem Acrylharz, das vorzugsweise ausgewählt ist aus der Gruppe von Poly(meth)acrylaten, Poly(C₁₋₄)dialkylamino-(C₁₋₄)alkyl(meth)acrylaten und/oder Copolymeren davon oder einem Gemisch von wenigstens zwei der zuvor genannten Polymere und/oder dass die wasserunlöslichen Polymere Zellulosederivate sind, vorzugsweise Alkylzellulose und besonders bevorzugt Ethylzellulose, oder Zelluloseester und/oder dass das Wachs Carnaubawachs, Bienenwachs, Glycerinmonostearat, Glycerinmonobehenat, Glycerinditripalmitostearat, mikrokristallines Wachs oder ein Gemisch aus wenigstens zwei dieser Bestandteile ist.

13. Pharmazeutische Formulierung nach einem oder mehreren der Ansprüche 11 bis 12, **dadurch gekennzeichnet, dass** Polymere in Kombination mit einem oder mehreren Weichmachern verwendet worden sind.

14. Pharmazeutische Formulierung nach einem oder mehreren der Ansprüche 10 bis 13, **dadurch gekennzeichnet, dass** neben der Form für anhaltende Freisetzung wenigstens einer der Wirkstoffbestandteile (A) oder (B) nicht in einer Retard-Form vorliegt.

## Revendications

1. Combinaison de substances actives qui comporte :
(A) du 6-chloro-N-(3-(2-(diméthylamino)éthyl)-1H-indol-5-yl)imidazo[2,1-b]thiazole-5-sulfonamide, et
(B) du donépézil ou un sel, racémique ou énantiomère de celui-ci.

2. Combinaison selon la revendication 1, **caractérisée en ce que** le composant (B) est de l'hydrochlorure de donépézil.

3. Combinaison selon l'une ou plusieurs des revendications 1 ou 2, **caractérisée en ce qu'**elle comprend entre 1 et 99% en masse du composant (A) et entre 99 et 1% en masse du composant (B), de préférence de 85 à 95% en masse du composant (A) et de 15 à 5% en masse du composant (B), en se référant dans chaque cas à la masse totale des deux composants (A) et (B).

4. Combinaison de substances actives selon l'une quelconque des revendications 1 à 3 pour son utilisation comme médicament.

5. Combinaison de substances actives selon l'une quelconque des revendications 1 à 3 pour son utilisation comme médicament pour l'inhibition simultanée de l'acétylcholinestérase et la régulation du récepteur 5-HT₆.

6. Combinaison de substances actives selon l'une quelconque des revendications 1 à 3 pour son utilisation comme médicament pour la régulation de l'appétit, pour le maintien, l'augmentation ou la réduction du poids corporel, pour la prévention et/ou le traitement de désordres liés à l'ingestion de nourriture, de préférence pour la prévention et/ou le traitement de l'obésité, l'anorexie, la cachexie, la boulimie, le diabète, de préférence le diabète de type II (diabète sucré non insulinodépendant), ou pour la prévention et/ou le traitement de désordres du tractus gastro-intestinal, de préférence du syndrome du colon irritable, pour la prévention et/ou le traitement du syndrome métabolique, des désordres du système nerveux périphérique, des désordres du système nerveux central, de l'arthrite, de l'épilepsie, de l'anxiété, la panique, la dépression, les désordres cognitifs, les désordres mémoriels, les maladies cardiovasculaires, les processus de démence sénile comme la maladie d'Alzheimer, la maladie de Parkinson et/ou la chorée de Huntington, la schizophrénie, la psychose, l'hyperkinésie infantile (désordre du déficit de l'attention et de l'hyperactivité), la douleur, le syndrome d'hypertension, les maladies inflammatoires, les maladies immunologiques ou pour l'amélioration de la cognition.

7. Formulation pharmaceutique, **caractérisée en ce qu'**elle comporte une combinaison de substances actives selon l'une ou plusieurs des revendications 1 à 3 et éventuellement un ou plusieurs adjuvants pharmacologiquement acceptables.

8. Formulation pharmaceutique selon la revendication 7, **caractérisée en ce qu'**elle est présente sous des formes pharmaceutiques solides telles que des tablettes, des tablettes de chewing-gum, des chewing-gums, des dragées, des capsules, des suppositoires, des préparations en poudre, des systèmes thérapeutiques transdermiques, des systèmes thérapeutiques transmuqueux, ou sous des formes pharmaceutiques liquides et semi-liquides comme des gouttes ou des jus, des sirops, des solutions, des émulsions, des suspensions, de préférences sous forme de tablettes, capsules, gouttes ou solution, ou sous forme de particules multiples de préférence des microtablettes, des microcapsules, des microsphéroïdes, des granulés, des cristaux ou des pastilles, éventuellement compactés en une tablette, contenus dans une capsule ou mis en suspension dans un liquide approprié.

9. Formulation pharmaceutique selon les revendications 7 ou 8, **caractérisée en ce qu'**elle est destinée à une application orale, intraveineuse, intramusculaire, sous-cutanée, intrathécale, péridurale, buccale, sublinguale, pulmonaire, rectale, transdermique, nasale ou intracérébroventriculaire, de préférence orale ou intraveineuse.

10. Formulation pharmaceutique selon les revendications 7 ou 8, **caractérisée en ce qu'**au moins un des composants de la combinaison de substances actives (A) ou (B) est présent au moins partiellement sous une forme à libération soutenue.

11. Formulation pharmaceutique selon la revendication 10, **caractérisée en ce que** le médicament a au moins un revêtement ou une matrice comprenant au moins un matériau à libération prolongée, de préférence le matériau à libération prolongée est basé sur un polymère naturel, synthétique ou semi-synthétique éventuellement modifié, non soluble dans l'eau, ou une cire ou une graisse naturelle ou un alcool gras ou un acide gras semi-synthétique ou synthétique, ou sur un mélange d'au moins deux de ces composants mentionnés ci-dessus.

12. Formulation pharmaceutique selon la revendication 11, **caractérisée en ce que** le polymère non soluble dans l'eau est basé sur une résine acrylique, qui est de préférence choisie dans le groupe composé des poly (méthyle) acrylates, poly (C₁₋₄)dialkylamino(C₁₋₄) alkyl(méthyle)acrylates et/ou des copolymères de ceux-ci ou un mélange d'au moins deux des polymères mentionnés ci-dessus et/ou les polymères non solubles dans l'eau sont des dérivés de cellulose, de préférence de l'alkyle de cellulose et de préférence encore de l'éthyle de cellulose, ou des esters de cellulose et/ou la cire est de cire de carnauba, de la cire d'abeille, du monostéarate de glycérol, du monobéhénate de glycérol, du ditripalmitostéarate de glycérol, de la cire microcristalline ou un mélange d'au moins deux de ces composants.

13. Formulation pharmaceutique selon l'une ou plusieurs des revendications 11 à 12, **caractérisé en ce que** les polymères ont été utilisés en combinaison avec un ou plusieurs plastifiants.

14. Formulation pharmaceutique selon l'une ou plusieurs des revendications 10 à 13, **caractérisée en ce qu'**en plus de la forme à libération prolongée, au moins un des composants de la substance active (A) ou (B) est présent sous une forme à libération non prolongée.
